# FASCICULE DE BREVET EUROPEEN

(11) **EP 1 988 838 B1**
(45) Date de publication et mention de la délivrance du brevet: **05.08.2015**
(21) Numéro de dépôt: 07731669.3
(22) Date de dépôt: 27.02.2007
(51) Int. Cl.: A61B 17/70

(54) **IMPLANT INTERVERTEBRAL**
ZWISCHENWIRBELIMPLANTAT
INTERVERTEBRAL IMPLANT

(30) Priorité: 28.02.2006 FR 0650687
(43) Date de publication de la demande: 12.11.2008
(73) Titulaire: ZIMMER SPINE, 33000 Bordeaux (FR)
(72) Inventeur: PASQUET, Denis, F-33360 Quinsac (FR); SENEGAS, Jacques, F-33700 Merignac (FR); LE COUEDIC, Régis, F-78570 Andresy (FR)
(74) Mandataire: Venner Shipley LLP
(86) Numéro de dépôt international: PCT/FR2007/050852
(87) Numéro de publication internationale: WO 2007/099258

(56) Documents cités:
- WO-A-02/051326
- WO-A-02/071960
- FR-A- 2 870 107
- FR-A1- 2 799 640
- US-A1- 2001 016 743

## Description

La présente invention a pour objet un implant intervertébral constitué par une cale destinée à être placée entre les apophyses épineuses de deux vertèbres consécutives et des liens pour solidariser les extrémités de la cale avec lesdites apophyses. Plus particulièrement, l'invention concerne un tel implant conçu pour être mis en place latéralement par rapport au plan défini par le rachis du patient auquel l'implant doit être mis.

De tels implants pourvus de cales intervertébrales sont notamment décrits dans la demande de brevet français 01 03362 et dans le brevet français 2870107 au nom de la demanderesse.

La mise en place d'une telle cale soulève certains problèmes liés à la pratique chirurgicale. En effet, un ligament dit supra-épineux relie tous les sommets des apophyses épineuses entre eux. Pour mettre en place la cale, il faut déplacer ce ligament. Pratiquement, on le détache des deux apophyses concernées et on l'écarte au moyen d'un instrument chirurgical approprié. Pour détacher le ligament des apophyses, on utilise un scalpel. Une fois que la cale est mise en place, on recoud le ligament aux épineuses après avoir pratiqué une petite ouverture dans ces dernières pour placer le fil de couture.

L'inconvénient majeur de cette pratique chirurgicale est qu'en touchant au ligament pour le détacher puis l'écarter, on lui fait perdre ses propriétés mécaniques. De plus, toutes ces opérations nécessitent du temps qui augmente la durée globale de l'intervention chirurgicale.

Le plus souvent, lorsqu'il faut procéder à l'ablation totale du disque naturel intervertébral, il est nécessaire d'avoir accès axialement à ce disque et donc la pratique chirurgicale décrite ci-dessus ne peut être évitée.

Il existe cependant au moins un cas où la situation est différente. C'est le cas où il faut procéder à l'ablation d'une hernie discale, intervention qui ne nécessite que l'accès à un côté du massif épineux. Dans ce cas, il serait donc particulièrement intéressant de disposer d'un implant intervertébral dans lequel la cale peut être aisément mise en place par voie latérale entre les apophyses épineuses puisque l'intervention chirurgicale proprement dite ne nécessite elle-même que cette voie d'accès.

Cela n'est pas possible avec les cales des implants intervertébraux connus car les ailes qui entourent le logement destiné à recevoir l'apophyse épineuse sont d'une hauteur relativement importante, typiquement au moins 5 mm. En effet, une telle hauteur d'aile imposerait une distraction des deux vertèbres concernées inacceptable.

Un objet de la présente invention est de fournir un implant intervertébral destiné à être disposé entre les apophyses épineuses qui puisse être mis en place latéralement sans présenter l'inconvénient mentionné ci dessus.

Pour atteindre ce but, selon l'invention, l'implant intervertébral destiné à être disposé entre les apophyses épineuses de deux vertèbres adjacentes comprend :
- une cale intervertébrale formée d'une seule pièce comprenant un corps ayant une direction longitudinale, présentant selon cette direction une première face d'insertion et une deuxième face et deux évidements disposés aux extrémités du corps selon sa direction longitudinale, chaque évidement étant défini par une première extension prolongeant ladite face d'insertion, une deuxième extension prolongeant la deuxième face du corps et une paroi de fond ; et
- deux moyens formant lien de fixation, chaque moyen formant lien présentant une première extrémité solidaire de ladite première extension du corps et une deuxième extrémité fixable sur ladite deuxième face pour entourer partiellement une apophyse épineuse et la maintenir dans un évidement.

L'implant se caractérise en ce que chaque première extension du corps a une hauteur par rapport à la paroi de fond de l'évidement au plus égale à celle de la deuxième extension qui lui est associée et dont la hauteur est comprise entre 1 et 3 mm.

On comprend que grâce au fait que chaque première extension de la cale définissant les évidements supérieur et inférieur présente une hauteur réduite par rapport à celle des implants connus, il est beaucoup plus aisé de mettre en place latéralement la cale entre les apophyses épineuses puisque l'écartement nécessaire entre ces apophyses est réduit.

Les inventeurs ont mis en évidence que cette hauteur réduite ne perturbait pas le maintien de l'apophyse dans le logement grâce à l'action des ligaments.

Ces caractéristiques permettent d'avoir des deuxièmes extensions plus hautes, ce qui favorise le maintien de l'apophyse dans le logement de la cale, notamment en coopération avec les liens de fixation.

De préférence, la hauteur de la première extension du corps de la cale est inférieure à 80 % ou préférentiellement 60% de la hauteur de la deuxième extension, ce qui permet effectivement de faciliter la mise en place de la cale par voie latérale puisqu'on limite ainsi l'écartement nécessaire entre les épineuses.

De préférence également, la hauteur de la première extension du corps de la cale est supérieure à 50 %, ou préférentiellement 30%, de la hauteur de la deuxième extension, ce qui assure que les apophyses épineuses seront effectivement mécaniquement maintenues dans les évidements définis par les paires d'extension du corps de la cale.

De préférence, l'implant comprend en outre des moyens de blocage des deuxièmes extrémités des moyens formant lien, lesdits moyens de blocage étant fixables de manière amovible sur la deuxième face dudit corps.

On comprend que cette disposition facilite le blocage et la tension des liens pour les chirurgiens après que ces liens aient été mis en place pour entourer les apophyses épineuses.

De préférence encore, la face d'insertion du corps de la cale est une portion de surface cylindrique dont les génératrices sont parallèles aux génératrices définissant les parois internes des évidements.

Cette caractéristique facilite l'insertion latérale de la cale entre les deux apophyses épineuses en raison de la forme de la face d'insertion et de sa forme cylindrique.

D'autres caractéristiques et avantages de l'invention apparaîtront mieux à la lecture de la description qui suit d'un mode de réalisation de l'invention donné à titre d'exemple non limitatif. La description se réfère aux figures annexées, sur lesquelles :
- la figure 1A est une vue en perspective de la cale dans laquelle l'organe de blocage des liens n'est pas solidarisé avec le corps de la cale ;
- la figure 1B est une vue similaire montrant l'organe de blocage des liens clipsé sur le corps de la cale ;
- la figure 2 est une vue en perspective de la cale illustrant le mode opératoire de mise en place de la cale entre les apophyses épineuses ; et
- la figure 3 est une vue en coupe longitudinale d'un mode préféré de réalisation de l'organe de blocage des liens.

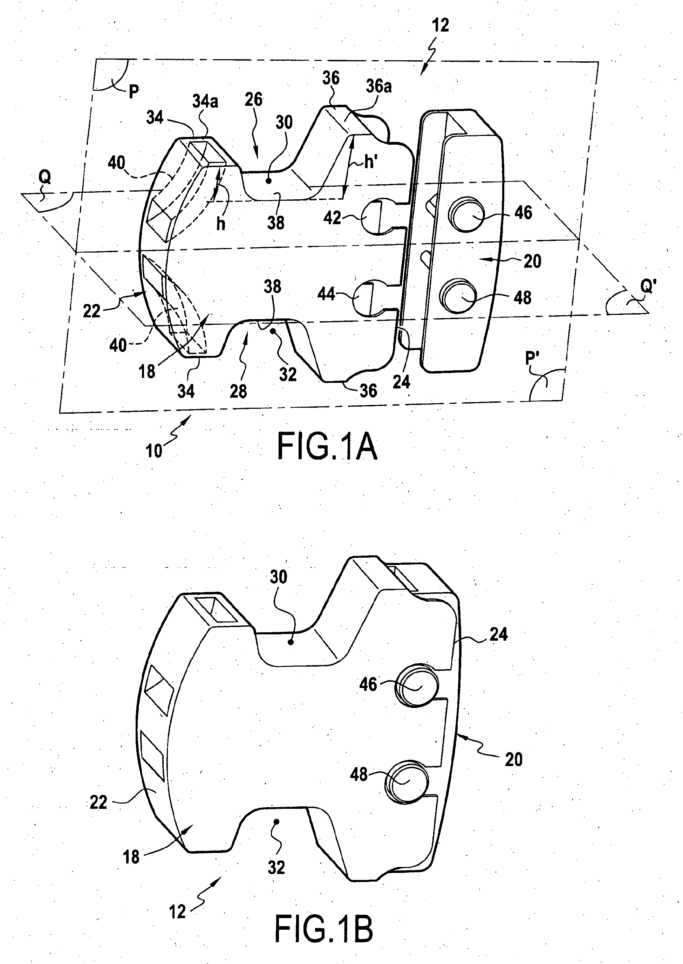

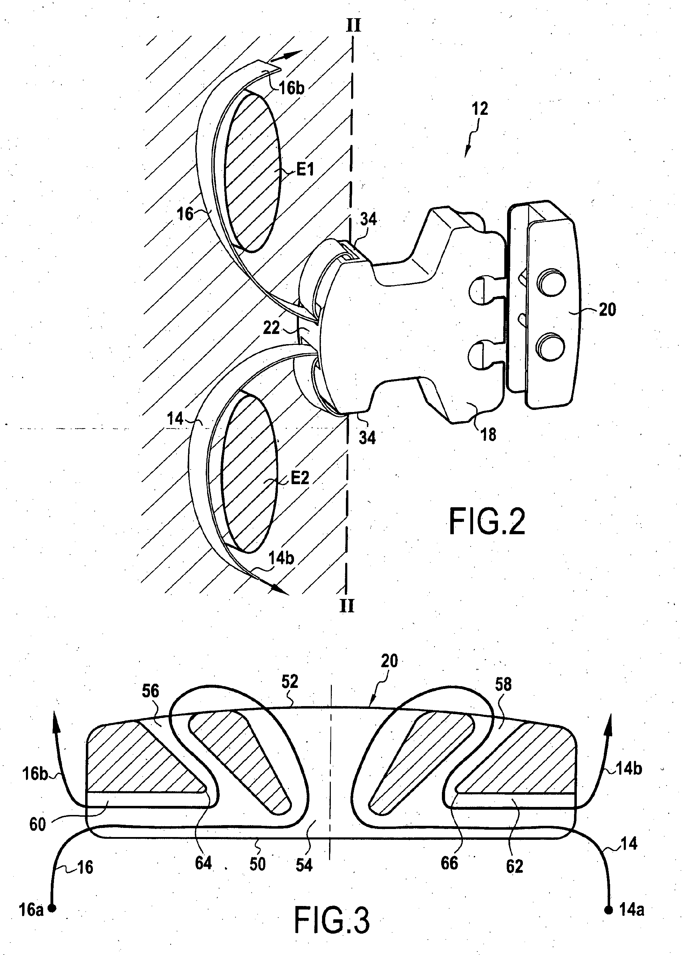

En se référant tout d'abord aux figures 1A et 1B, on va décrire la structure de la cale intervertébrale. L'implant intervertébral, qui est référencé 10, est constitué de préférence par une cale intervertébrale 12 et par deux liens de fixation 14 et 16 visibles sur les figures 2 et 3. La cale intervertébrale 12 est de préférence constituée par un corps 18, par exemple réalisé en PEEK, et par un organe de blocage des liens 20 qui, dans ce mode de réalisation, est amovible par rapport au corps de la cale. Il va cependant de soi qu'on ne sortirait pas de l'invention si les moyens de blocage des liens faisaient partie intégrante du corps 18 de la cale.

Le corps de cale 18 comporte un plan médian PP' par rapport auquel le corps est symétrique. Le corps 18 comporte une première face d'insertion 22 et une deuxième face opposée 24. Dans le mode de réalisation décrit, l'organe de blocage 20 est fixé sur la deuxième face 24. De préférence, la face d'insertion 22 a une forme de portion de surface cylindrique convexe dont les génératrices sont orthogonales au plan médian PP' et qui correspond sensiblement à une portion de surface cylindrique circulaire. Le corps de cale comporte un deuxième plan médian QQ' orthogonal au premier plan médian PP'.

A ces deux extrémités opposées 26 et 28, le corps de cale 18 présente de façon connue des logements respectivement référencés 30 et 32 destinés à recevoir les apophyses épineuses. Chaque logement est défini par deux extensions ou ailes 34 et 36, l'extension 34 prolongeant la face d'insertion 22 du corps de cale, les extensions 36 prolongeant la deuxième face 24 du corps de cale. Chaque logement 30, 32 comporte également un fond 38. Ainsi, chaque logement 30, 32 est limité par les faces internes des extensions 34 et 36 et par la paroi de fond 38. La paroi interne de chaque logement est une surface réglée dont les génératrices sont parallèles au deuxième plan médian QQ'.

Selon une caractéristique essentielle de l'invention, la hauteur h des premières extensions 34 proches de la face d'insertion de la cale ont une hauteur h qui est au plus égale à la hauteur h' des deuxièmes extensions 36 proches de la deuxième face 24 du corps de cale. Par hauteur des extensions, il faut entendre la distance selon le plan médian PP' qui sépare la paroi de fond 38 du logement de l'extrémité supérieure 34a, 36a des extensions.

La hauteur h' est, de préférence, la hauteur standard des extensions de corps de cale classiques, c'est à dire au moins égale à 5 mm. La hauteur h des premières extensions est, de préférence, inférieure à 80 %, ou de préférence encore 60%, de la hauteur h' des deuxièmes extensions pour faciliter l'insertion de la cale entre les apophyses épineuses. En revanche, de préférence également, la hauteur h est au moins égale à 30 %, ou préférentiellement 50 %, de la hauteur h' pour définir des logements 30, 32 de profondeur suffisante pour assurer le maintien mécanique des apophyses épineuses dans les logements.

De toutes manières, la hauteur h des premières extensions est comprise entre 3 mm et 1 mm.

Les premières extensions 34 comportent en outre un passage interne 40 qui débouche respectivement dans l'extrémité supérieure 34a de l'extension et dans la phase d'insertion 22. Grâce à ce passage, la première extrémité 16a, 14a de chaque lien peut être rendue solidaire du corps de cale à proximité de sa face d'insertion 22.

Pour assurer la tension et le blocage en tension des liens 14 et 16, la cale est équipée, comme on l'a déjà indiqué, d'un organe de blocage 20 qui, dans le mode de réalisation considéré, peut être fixé de manière amovible sur la deuxième face 24 du corps de cale. Pour cela, le corps de cale à proximité de sa face 24 peut comporter deux paires d'évidements élastiquement déformables 42 et 44 aptes à coopérer avec des ergots de clipsage 46 et 48 ménagés dans les faces de l'organe de blocage 20 parallèles au plan médian PP'.

En se référant maintenant à la figure 3, on va décrire sommairement un mode préféré de réalisation de l'organe de blocage 20, cet organe de blocage étant décrit en détail dans la demande de brevet français 04 05064.

L'organe de blocage 20 est constitué par une pièce qui présente une première face 50 destinée à coopérer avec la deuxième face 24 du corps de la cale et une face opposée 52. Dans la pièce formant l'organe de blocage 20, est prévue une fente axiale 54 qui s'étend entre les faces 50 et 52 de l'organe de blocage et qui a une forme évasée depuis la face 50 vers la face 52. La pièce constituant l'organe de blocage comporte également deux fentes latérales symétriques respectivement référencées 56 et 58. Enfin, cette pièce définit des passages 60 et 62 pour permettre le passage des liens 14 et 16. Chaque passage 60 et 62 définit avec les fentes latérales 56, 58 une arête de frottement 64 et 66.

Sur la figure 3, on a montré le trajet de chaque lien 14 et 16 dans les différentes fentes de l'organe de blocage 20. Lorsque l'organe de blocage 20 est verrouillé sur le corps de cale, en exerçant une traction sur les extrémités libres 16b et 14b des liens 16 et 14, on obtient une tension de ces liens et un autoblocage de ceci assurant le maintien des apophyses épineuses dans les logements 30 et 32 de la cale.

En se référant maintenant à la figure 2, on va décrire la technique chirurgicale pour la mise en place de l'implant 10.

Pour la mise en place de l'implant, le chirurgien procède aux étapes suivantes.

Il réalise une incision II légèrement à droite de l'axe du rachis du patient. La partie hachurée représente la zone masquée par la peau du patient. Ensuite, le chirurgien écarte les muscles de droite vers la droite d'une distance de l'ordre de 3 à 4 cm. Dans l'étape suivante, il procède à l'enlèvement du ligament intra-épineux. Ces opérations ayant été réalisées, le chirurgien introduit dans l'incision les extrémités libres des liens 14 et 16 et les fait passer autour des apophyses épineuses E1 et E2. Il ramène ces extrémités libres vers l'extérieur de l'incision. Le chirurgien insère alors le corps de la cale dans l'incision entre les apophyses épineuses en présentant d'abord la face d'insertion 22. Comme on l'a déjà expliqué, du fait que les extensions 34 correspondant à la face d'insertion du corps de cale ont une hauteur réduite, l'écartement nécessaire des apophyses épineuses est réduit. En outre, la forme cylindrique de la face d'insertion facilite la mise en place de la cale entre les apophyses. Lorsque les apophyses épineuses sont entrées dans les logements du corps de cale, le chirurgien insère les extrémités libres des liens dans les différents passages de l'organe de blocage 20 et provoque le clipsage de l'organe de blocage 20 sur la face 24 du corps de cale. Il lui suffit alors d'exercer une traction convenable sur les extrémités libres 16b, 14b des deux liens pour assurer un serrage auto-bloquant des liens sur les apophyses épineuses.

De préférence, la face d'insertion 22 du corps est une surface cylindrique dont les génératrices sont parallèles aux génératrices définissant les parois internes des évidements 30, 32.

De préférence également, le corps 18 comprend, selon sa direction longitudinale, une partie médiane et deux parties d'extrémité dans laquelle sont définis lesdits évidements 30, 32, les parties d'extrémité présentant une rigidité supérieure à celle de la paroi médiane.

De préférence encore, chaque moyen formant lien 12, 14 est constitué par une bande d'un matériau souple.

## Revendications

1. Implant intervertébral destiné à être disposé entre les apophyses épineuses de deux vertèbres adjacentes comprenant :
- une cale intervertébrale (12) formée d'une seule pièce comprenant un corps (18) ayant une direction longitudinale, présentant selon cette direction une première face d'insertion (22) et une deuxième face (24) et deux évidements (30, 32) disposés aux extrémités du corps selon sa direction longitudinale, chaque évidement étant défini par une première extension (34) prolongeant ladite face d'insertion, une deuxième extension (36) prolongeant la deuxième face du corps et une paroi de fond (38) ; et
- deux moyens formant lien de fixation (14, 16), chaque moyen formant lien présentant une première extrémité solidaire de ladite première extension du corps (34) et une deuxième extrémité fixable sur ladite deuxième face (24) pour entourer partiellement une apophyse épineuse et la maintenir dans un évidement,
**caractérisé en ce que** chaque première extension (34) du corps (18) a une hauteur par rapport à la paroi de fond de l'évidement qui est au plus égale à celle de la deuxième extension qui lui est associée et qui est comprise entre 1 mm et 3 mm.

2. Implant selon la revendication 1, **caractérisé en ce que** la hauteur de la première extension (34) du corps est inférieure à 80 %, de préférence 60 %, de la hauteur de la deuxième extension.

3. Implant selon l'une quelconque des revendications 1 et 2, **caractérisé en ce que** la hauteur de la première extension (34) du corps est supérieure à 30 %, de préférence 50 %, de la hauteur de la deuxième extension.

4. Implant selon l'une quelconque des revendications 1 à 3, **caractérisé en ce que** la cale présente un plan de symétrie orthogonal (QQ') à sa direction longitudinale, et **en ce que** ladite face d'insertion (22) présente une forme convexe symétrique par rapport audit plan de symétrie.

5. Implant selon l'une quelconque des revendications 1 à 4, **caractérisé en ce qu'**il comprend en outre des moyens de blocage (20) des deuxièmes extrémités des moyens formant lien (14, 16), lesdits moyens de blocage étant fixable de manière amovible sur la deuxième face (24) dudit corps.

6. Implant selon la revendication 5, **caractérisé en ce que** lesdits moyens de blocage (20) comprennent, à chacune de leurs extrémités selon la direction longitudinale du corps, un système auto-bloquant de la deuxième extrémité d'un des moyens formant lien.

7. Implant selon l'une quelconque des revendications 1 à 5, **caractérisé en ce que** la paroi interne de chaque évidement (30, 32) est une surface cylindrique dont les génératrices sont orthogonales à la direction longitudinale du corps.

8. Implant selon les revendications 4 et 7, **caractérisé en ce que** ladite face d'insertion (22) du corps est une surface cylindrique dont les génératrices sont parallèles aux génératrices définissant les parois internes des évidements (30, 32).

9. Implant selon l'une quelconque des revendications 1 à 8, **caractérisé en ce que** ledit corps (18) comprend, selon sa direction longitudinale, une partie médiane et deux parties d'extrémité dans laquelle sont définis lesdits évidements (30, 32), les parties d'extrémité présentant une rigidité supérieure à celle de la partie médiane.

10. Implant selon l'une quelconque des revendications 1 à 9, **caractérisé en ce que** chaque moyen formant lien (14, 16) est constitué par une bande d'un matériau souple.

## Patentansprüche

1. Zwischenwirbel-Implantat zur Anordnung zwischen den Dornfortsätzen von zwei benachbarten Wirbeln, umfassend:
- einen Zwischenwirbelkeil (12), der einstückig ausgebildet ist und einen Körper (18) mit einer Längsrichtung umfasst, der in dieser Richtung eine erste Einsatzfläche (22) und eine zweite Fläche (24) und zwei Aussparungen (30, 32) aufweist, die an den Enden des Körpers in dessen Längsrichtung angeordnet sind, wobei jede Aussparung durch eine erste Verlängerung (34), welche die Einsatzfläche verlängert, eine zweite Verlängerung (36), welche die zweite Fläche des Körpers verlängert, und eine Bodenwand (38) definiert wird; und
- zwei Mittel, die eine Befestigungsverbindung (14, 16) bilden, wobei jedes die Verbindung bildende Mittel ein erstes Ende aufweist, das mit der ersten Verlängerung des Körpers (34) fest verbunden ist, und ein zweites Ende aufweist, das an der zweiten Fläche (24) befestigbar ist, um einen Dornfortsatz teilweise zu umgeben und ihn in einer Aussparung zu halten,
**dadurch gekennzeichnet, dass** jede erste Verlängerung (34) des Körpers (18) eine Höhe relativ zu der Bodenwand der Ausnehmung hat, die höchstens gleich derjenigen der zweiten Verlängerung ist, die ihr zugeordnet ist, und die zwischen 1 mm und 3 mm beträgt.

2. Implantat nach Anspruch 1, **dadurch gekennzeichnet, dass** die Höhe der ersten Verlängerung (34) des Körpers kleiner als 80 %, vorzugsweise 60 % der Höhe der zweiten Verlängerung ist.

3. Implantat nach einem der Ansprüche 1 und 2, **dadurch gekennzeichnet, dass** die Höhe der ersten Verlängerung (34) des Körpers größer als 30 %, vorzugsweise 50 % der Höhe der zweiten Verlängerung ist.

4. Implantat nach einem der Ansprüche 1 bis 3, **dadurch gekennzeichnet, dass** der Keil eine Symmetrieebene (QQ') aufweist, die zu seiner Längsrichtung orthogonal ist, und dass die Einsatzfläche (22) eine konvexe Form aufweist, die relativ zu der Symmetrieebene symmetrisch ist.

5. Implantat nach einem der Ansprüche 1 bis 4, **dadurch gekennzeichnet, dass** es ferner Mittel zum Blockieren (20) der zweiten Enden der die Verbindung bildenden Mittel (14, 16) umfasst, wobei die Blockiermittel an der zweiten Fläche (24) des Körpers lösbar befestigt sind.

6. Implantat nach Anspruch 5, **dadurch gekennzeichnet, dass** die Blockiermittel (20) an jedem ihrer Enden in der Längsrichtung des Körpers ein selbsthemmendes System des zweiten Endes eines der die Verbindung bildenden Mittel umfasst.

7. Implantat nach einem der Ansprüche 1 bis 5, **dadurch gekennzeichnet, dass** die Innenwand jeder Ausnehmung (30, 32) eine zylindrische Fläche ist, deren Mantellinien zu der Längsrichtung des Körpers orthogonal sind.

8. Implantat nach Anspruch 4 und 7, **dadurch gekennzeichnet, dass** die Einsatzfläche (22) des Körpers eine zylindrische Fläche ist, deren Mantellinien zu den Mantellinien, welche die Innenwände der Ausnehmungen (30, 32) definieren, parallel sind.

9. Implantat nach einem der Ansprüche 1 bis 8, **dadurch gekennzeichnet, dass** der Körper (18) in seiner Längsrichtung einen Mittelteil und zwei Endteile umfasst, in denen die Aussparungen (30, 32) definiert sind, wobei die Endteile eine Steifigkeit aufweisen, die größer ist als die der Mittelteile.

10. Implantat nach einem der Ansprüche 1 bis 9, **dadurch gekennzeichnet, dass** jedes die Verbindung bildende Mittel (14, 16) von einem Band aus weichem Material gebildet ist.

## Claims

1. Intervertebral implant intended to be placed between the spinous processes of two adjacent vertebrae, comprising:
- an intervertebral spacer (12) formed in one piece comprising a body (18) having a longitudinal direction, having in said direction a first insertion face (22) and a second face (24), and two recesses (30, 32) arranged at the ends of the body in the longitudinal direction thereof, each recess being defined by a first extension (34) extending said insertion face, by a second extension (36) extending the second face of the body, and by a bottom wall (38); and
- two means forming fastener ties (14, 16), each tie-forming means having a first end which is rigidly connected to said first extension of the body (34), and a second end which can be fastened to said second face (24) so as surround a spinous process in part and hold said process in a recess,
**characterised in that** each first extension (34) of the body (18) has a height relative to the bottom wall of the recess that is at least equal to that of the second extension associated therewith, and that lies in the range of between 1 mm and 3 mm.

2. Implant according to claim 1, **characterised in that** the height of the first extension (34) of the body is less than 80 %, preferably 60 %, of the height of the second extension.

3. Implant according to either claim 1 or claim 2, **characterised in that** the height of the first extension (34) of the body is greater than 30 %, preferably 50 %, of the height of the second extension.

4. Implant according to any of claims 1 to 3, **characterised in that** the spacer has a plane of symmetry (QQ') which is orthogonal to its longitudinal direction, and **in that** said insertion face (22) has a convex shape that is symmetrical about said plane of symmetry.

5. Implant according to any of claims 1 to 4, **characterised in that** it further comprises blocking means (20) for blocking the second ends of the tie-forming means (14, 16), it being possible for said blocking means to be fastened in releasable manner to the second face (24) of said body.

6. Implant according to claim 5, **characterised in that** said blocking means (20) comprise, at each of their ends in the longitudinal direction of the body, a system for automatically blocking the second end of one of the tie-forming means.

7. Implant according to any of claims 1 to 5, **characterised in that** the inside wall of each recess (30, 32) is a cylindrical surface of which the generatrices are orthogonal to the longitudinal direction of the body.

8. Implant according to claims 4 and 7, **characterised in that** said insertion face (22) of the body is a cylindrical surface of which the generatrices are parallel to the generatrices defining the inside walls of the recesses (30, 32).

9. Implant according to any of claims 1 to 8, **characterised in that** said body (18) comprises, in its longitudinal direction, a middle portion and two end portions in which said recesses (30, 32) are defined, the end portions having a rigidity that is greater than that of the middle portion.

10. Implant according to any of claims 1 to 9, **characterised in that** each tie-forming means (14, 16) is formed by a strip of flexible material.
